# EUROPÄISCHE PATENTSCHRIFT

(11) **EP 1 120 468 B1**
(45) Veröffentlichungstag und Bekanntmachung des Hinweises auf die Patenterteilung: **24.02.2010**
(21) Anmeldenummer: 01100642.6
(22) Anmeldetag: 11.01.2001
(51) Int. Cl.: C12Q 1/32

(54) **Stabilisierte Coenzym-Lösungen und deren Verwendung zur Bestimmung von Dehydrogenasen bzw. deren Substrate**
Stabilised coenzyme solutions and their use for the determination of dehydrogenases or their substrates
Solutions stabilisés de co-enzymes et leur utilisation pour la détermination des déshydrogénases ou leurs substrates

(30) Priorität: 15.01.2000 DE 10001529
(43) Veröffentlichungstag der Anmeldung: 01.08.2001
(73) Patentinhaber: Roche Diagnostics GmbH, 68305 Mannheim (DE); F. Hoffmann-La Roche AG, 4070 Basel (CH)
(72) Erfinder: Zielenski, Ralf, 83671 Benediktbeuern (DE)

(56) Entgegenhaltungen:
- EP-A- 0 640 686
- EP-A- 0 666 323
- WO-A-97/19190
- US-A- 4 271 264
- US-A- 4 310 624

## Beschreibung

Die Erfindung betrifft stabilisierte wäßrige Lösungen eines Coenzyms für wasserstoffübertragende Enzyme sowie deren Verwendung zur Bestimmung eines entsprechenden Analyten (Substrates) in reduzierter Form oder der Enzymaktivität einer entsprechenden Dehydrogenase. Die stabilisierte Lösung enthält eine organische Verbindung bzw. entsprechende Salze mit einem pKa-Wert zwischen 1,5 und 6,0 und/oder ein Hydroxylaminderivat.

Die Bestimmung von Enzymaktivitäten (od. Substratkonzentrationen), insbesondere in Blut-Serum oder -Plasma, spielt eine wichtige Rolle in der klinisch chemischen Diagnostik. Häufig werden dazu Testverfahren verwendet, die auf der Reduktion von Nicotinamid-adenin-dinucleotid ("NAD") bzw. Nicotinamid-adenin-dinucleotid-phosphat ("NADP") und der photometrischen Erfassung der dabei erfolgenden Änderung des Absorptionsverhaltens im ultravioletten Wellenlängenbereich (λ=334, 340 oder 365 nm) beruhen. Bei der Wahl geeigneter Testbedingungen ist diese Änderung linear proportional zu der zu bestimmenden Enzymaktivität (bzw. Substratkonzentration).

Zur Bestimmung der Enzymaktivität von beispielsweise Lactatdehydrogenase (LDH, E.C.1.1.1.27) wird heute allgemein das in Eur. J. Clin. Chem. Clin. Biochem. 31, 897 (1994) und Eur. J. Clin. Chem. Clin. Biochem. 32, 639 (1994) beschriebene Verfahren empfohlen. Das Testprinzip sieht die Oxidation von Lactat zu Pyruvat unter gleichzeitiger Reduktion eines Coenzyms wie NAD oder NADP zu NADH bzw. NADPH vor. Eine solche - wie hier beispielsweise von LDH katalysierte - Umsetzung findet im alkalischen Medium (pH 9,4) statt. Diese Instabilität äußert sich in einem verhältnismäßig raschen Extinktionsanstieg (dem sog. Reagenzleerwert) im, die Messung betreffenden Wellenlängenbereich, so daß bereits nach kurzer Zeit (3 Monate) auch bei Kühllagerung (2° bis 8°C) die Reagenzkombination unbrauchbar wird. Dieses Problem stellt sich im besonderen Maße für die Herstellung gebrauchsfertiger, langzeitstabiler Flüssigreagenzien, die dem Anwender eine möglichst einfache und sichere Durchführung von Analysen in der täglichen Routine ermöglichen sollen.

Ein Verfahren zur Stabilisierung von wäßrigen Coenzymen unter Verwendung von Chelatbildnern und Aziden ist aus JP 84/82398 bekannt. Nachteilig an diesem Verfahren ist jedoch, daß der Zusatz von Azid erforderlich ist, da Azid zum einen heute als Cancerogen eingestuft ist und zudem auf viele Enzyme eine inhibierende Wirkung hat.

Ferner ist bekannt, Coenzmlösungen durch den Zusatz von Schwermetallsalzen, beispielsweise in Form von Kupfer (II)-Ionen, zu stabilisieren und dadurch einen Anstieg des Reagenzienleerwertes zu unterbinden (DE 195 43 493 bzw. EP 0 804 610). Bei längerer Lagerzeit bzw. bei höheren Lagertemperaturen (bereits ab 10°C) können jedoch Abbauprodukte gebildet werden, die das zu bestimmende Dehydrogenase-Enzym inhibieren und somit zu niedrige Meßwerte bewirken. Ein über einen längeren Zeitraum (3 und mehr Monate) stabilisierbares Reagenz mit gleichbleibender Qualität, wodurch unter anderem wiederholte Kalibrierungen vermieden werden können, steht derzeit nicht zur Verfügung.

Der vorliegenden Erfindung liegt somit die Aufgabe zugrunde, ein verbessertes ein Coenzym für wasserstoffübertragende Enzyme aufweisendes, stabiles Flüssigreagenz, welches zur Bestimmung von Dehydrogenase-Aktivität bzw. entsprechender Substrate geeignet ist, zur Verfügung zu stellen.

Die Aufgabe wird gelöst durch eine wäßrige Lösung gemäß Anspruch 1.Als Alkylgruppen sind insbesondere solche Reste geeignet, die ein bis zehn Kohlenstoffatome aufweisen. Die Alkylgruppen können darüber hinaus geradkettig oder verzweigt sein. Als Arylgruppen kommen erfindungsgemäß substituierte oder unsubstituierte, gegebenenfalls über eine Alkenylgruppe, welche ein bis 8 Kohlenstoffatome aufweisen kann, gebundene, Phenylgruppen in Betracht. Insbesondere haben sich Stickstoffverbindungen der genannten allgemeinen Formel (I), d.h. insbesondere Hydroxylaminderivate wie Hydroxylamin, O- oder N-Alkylhydroxylamin mit einem bis sechs Kohlenstoffatomen oder O-Benzylhydroxylamin und davon abgeleitete Salze, wie z. B. Sulfate, Phosphate, oder Ammoniumsalze, erfindungsgemäß als geeignet erwiesen. Entsprechende Hydroxylaminderivate zeichnen sich zudem über eine komplexierende Wirkung gegenüber den Abbauprodukten des Coenzyms aus.

Die Stabilität der Lösungen kann zudem weiter verbessert werden, wenn zusätzlich ein Komplexbildner, d.h. ein Ligand, welcher zwei oder mehr Koordinationsstellen aufweist, in der Lösung enthalten ist. Es haben sich hier sowohl zweizähnige Liganden, wie z.B. Ethylendiamin und vier-bzw. mehrzähnige Liganden, wie Ethylendiamin-N, N, N, N-tertraessigsäure (EDTA) bzw. entsprechende Salze, insbesondere das Dinatriumsalz, Kronenether oder Kryptanden als vorteilhaft erwiesen. Dies entspricht einer Konzentration des Komplexbildners von ungefähr 0,5 bis 30 mM, vorzugsweise von 1,0 bis 5,0 mM.

Erfindungsgemäß zuzusetzende organische Verbindungen bzw. davon abgeleitete Salze mit einem pKa-Wert zwischen ca. 1,5 und 6,0 sind insbesondere solche organischen Säuren, die über eine komplexierende Wirkung verfügen und eine puffernde Wirkung im pH-Bereich von 1,0 bis 7,0 aufweisen, wie beispielsweise Citronensäure und davon abgeleitete wasserlösliche Salze.

Die Konzentrationen der erfindungsgemäß zuzusetzenden organischen Verbindungen, Salze oder Hydroxylaminderivate können in weiten Grenzen, d.h. zwischen ca. 0,001 und 1,0 M variieren. Als besonders geeignet haben sich für Citronensäure bzw. Citrat eine Konzentration von ca. 5 bis 200 mM erwiesen. In zahlreichen Fällen führten bereits ca. 50 mM Citronensäure bzw. Citrat zu dem gewünschten Effekt. Der bevorzugte Konzentrationsbereich für das erfindungsgemäß zusätzlich zu oder in Abwesenheit einer organischen Verbindung mit entsprechendem pKa-Wert zuzusetzende Hydroxylaminderivat liegt zwischen etwa 2 und 300 mM. Der pH-Wert der stabilisierten wäßrigen Lösung kann zwischen 1,0 und 7,0 betragen, wobei sich ein pH-Wert zwischen ca. 2,0 und 4,0 bzw. von ca. 3,0 als besonders vorteilhaft erwiesen hat.

Als besonders vorteilhaft hat sich darüber hinaus erwiesen, wenn ein Hydroxylaminderivat und gegebenenfalls zusätzlich ein Citratsalz in dem NAD bzw. NADP haltigen Reagenz enthalten sind, und gegebenenfalls in einem weiteren für die Bestimmung entsprechender wasserstoffübertragender Analyten erforderlichen Reagenz, welches insbesondere für die Bestimmung erforderliche Puffer, wie z. B. N-Methylglucamin (MEG), Substrate und gegebenenfalls weitere Hilfsstoffe enthält, zusätzlich Borsäure bzw. ein Boratsalz enthalten ist. Auch für diese besondere Ausführungsform gelten die oben angeführten Konzentrationsangaben. Darüber hinaus haben sich ca. 20 bis 200 mM für Citrat bzw. Citronensäure und ca. 10 bis 150 mM für das jeweilige Hydroxylaminderivat als besonders vorteilhaft erwiesen. Für das vorzugsweise der nicht NAD/NADP haltigen Substratlösung (sogenanntes Reagenz 1) zuzusetzende Borsäurederivat hat sich ein Konzentrationsbereich von ca. 50 bis 200 mM als besonders geeignet erwiesen.

Als Puffer sind prinzipiell solche Substanzen für das substrathaltige Reagenz geeignet, die eine gute Pufferkapazität zwischen ca. pH 8,5 und 10,0 aufweisen, wie beispielsweise die sogenannten Good-Puffer (Tricine, Bicine, TAPS, AMPSO, CHES, CAPSO, AMP, CAPS), Carbonate von Alkalimetallionen, MEG, TRIS sowie Phosphatpuffer. Auch Mischungen der genannten Puffersubstanzen haben sich für die erfindungsgemäße Lösung als geeignet erwiesen. Ferner hat sich als vorteilhaft erwiesen, wenn die Pufferkonzentration zwischen ca. 10 und 1000 mM, bevorzugt zwischen 200 und 600 mM beträgt. In der alkalischen Pufferlösung (Reagenz 1), die den Arbeits-pH-Wert maßgeblich bestimmt, hat sich zudem der Zusatz von Borsäure bzw. deren löslichen Salze und Derivate als vorteilhaft erwiesen. Die Konzentration entsprechender Borsäurekomponenten liegt bevorzugt zwischen etwa 50 und 200 mM, besonders bevorzugt bei etwa 100 m.

Als Coenzyme im Sinne der vorliegenden Erfindung kommen insbesondere NAD bzw. NADP, aber auch modifizierte Coenzyme, wie beispielsweise thioNAD(P) oder NHxDP (=Nicotinamidhypoxanthin-dinukleotidphosphat) in Betracht. Die Coenzyme können in einer Konzentration von ungefähr 1,0 bis 100 mM in der Reaktionsküvette vorliegen, bevorzugt ist hier ein Bereich von 5,0 bis 15,0 mM.

Die erfindungsgemäßen stabilisierten Coenzym-Lösungen, werden in Form von wäßrigen Lösungen verwendet. Das gebrauchsfertige Reagenz ist darüber hinaus aber auch als Granulat, Pulvermischung sowie als Lyophilisat über einen weiten Zeitraum hin stabil. So konnten bei Temperaturen von 2° bis 8°C innerhalb von 15 Monaten keinerlei Zersetzungserscheinungen des Reagenzes festgestellt werden. Unter Belastung, d.h. bei einer Temperatur von ca. 35°C für 2 Wochen oder der Behandlung bei ca. 42°C für fünf Tage konnte gezeigt werden, daß die Lösung mit einem oder mehreren erfindungsgemäßen Zusätzen qualitativ unverändert, d.h. stabil bleibt.

Ein weiterer Gegenstand der Erfindung ist, ein Verfahren zur Bestimmung eines wasserstoffübertragenden Analyten bzw. einer entsprechenden Dehydrogenase in Gegenwart eines wasserstoffaufnehmenden Coenzyms, wobei das Coenzym in einer stabilisierten wäßrigen Lösung wie oben beschrieben vorliegt.

Die Bestimmung erfolgt insbesondere in Proben biologischen Ursprungs wie beispielsweise Vollblut, Serum oder Plasma bzw. anderen menschlichen bzw. tierischen Quellen oder in pflanzlichen Extrakten. Die Probe kann mit physiologischer Kochsalzlösung aufbereitet werden. Als Kontrollwert dient in einem solchen Fall vorteilhafterweise eine 0,9% NaCl-Lösung.

Für den Fall, daß die Enzymaktivität einer Dehydrogenase wie beispielsweise Lactatdehydrogenase bestimmt werden soll, wird eine Substratlösung, beispielsweise eine Lactatlösung, in einer bei ca. pH 9,4 (37°C) puffernden Substanz(mischung) verwendet. Das Substrat kann dabei in den üblichen, dem Fachmann bekannten Konzentrationen eingesetzt werden, vorzugsweise in einem Bereich von 40 bis 80 mM.

Für die Bestimmung eines wasserstoffübertragenden Analyten wie beispielsweise Lactat, wird die jeweilige Dehydrogenase, wie beispielsweise LDH in einer zwischen pH 8,5 und 10,0 puffernden Substanz vorgelegt. In der Regel ist eine Menge an Dehydrogenase von ungefähr 70 bis 500 U/l, bevorzugt von 110 bis 220 U/l ausreichend. Die Bestimmung wird üblicherweise bei ca. 37°C durchgeführt.

Neben dem beispielhaft angeführten Lactat können in analoger Weise Glutamat bzw. Ammoniak, Alkohol, Glycerinaldehyd-3-phosphat, Glucose oder andere Parameter, welche durch eine geeignete Coenzymabhängige Dehydrogenase umgesetzt werden können, bestimmt werden. Entsprechendes gilt für die Bestimmung der Enzymaktivität solcher Dehydrogenasen.

Ein weiterer Gegenstand der Erfindung ist ein sogenannter Testkit zur Ausführung der Enzym-bzw. Analytbestimmung. Der Kit besteht im wesentlichen aus zwei Teilreagenzien. Das erste Reagenz beinhaltet - für den Fall der Bestimmung der Aktivität einer Dehydrogenase - einen wasserstoffübertragenden Analyten (Substrat) in einem geeigneten zwischen pH 8,5 und 10,0 puffernden System. Das zweite Reagenz enthält ein Coenzym für wasserstoffübertragende Enzyme, wie beispielsweise NAD bzw. NADP, sowie eine organische Verbindung mit einem pKa-Wert zwischen 1,5 und 6,0 und/oder ein erfindungsgemäßes Hydroxylaminderivat auf. Darüber hinaus kann das zweite Reagenz weitere Hilfsstoffe, wie z. B. Schwermetallsalze oder einen Komplexbildner enthalten. Entsprechend ist für den Fall der Analyt- bzw. Substratbestimmung, wie beispielsweise von Lactat, zu verfahren.

### Abkürzungen

- AMP =: 2-Amino-2-methyl-1-propanol
- AMPSO =: 3-[(1,1-Dimethyl-2-hydroylethyl)amino-2-hydroxypropansulfonsäure
- Bicine =: N,N-Bis[2-Hydroxyethyl] glycin
- CAPS =: 3-[Cyclohexylamino]-1-propansulfonsäure
- CAPSO =: 3-[Cyclohexylamino]-2-hydroxy-1-propansulfonsäure
- CHES =: 2-[N-Cyclohexylamino] ethansulfonsäure
- MEG =: N-Methylglucamin
- TAPS =: N-Tris[Hydroxymethyl] methyl-3-aminopropansulfonsäure
- Tricine =: N-Tris[Hydroxymethyl] methylglycerin
- TRIS =: 2-Amino-2-(hydroxymethyl)-1,3-propanol

Die folgenden Beispiele erläutern die Erfindung weiter:

### Beispiel 1

Reagenz 1: N-Methylglucamin 390 mmol/l pH 9,4 (37°C); Lithium-L-Lactat 60 mmol/l.

Reagenz 2: NAD(P) 60 mmol/1 als Lyophilisat, Pulvermischung, Granulat oder wäßrige Lösung.

Inkubationstemperatur: 37 ± 0,1°C; Meßwellenlänge 340 ± 2 nm; Schichtdicke 7 mm;

Vorinkubation: 5 Minuten; Lag phase: 2 Minuten; Meßzeit: 2 Minuten.

Reagenz 1 = 250 µl; Reagenz 2 = 50 µl; Probe 7 µl NaCl-Lösung (0,9% w/v).

Folgende Bestimmungen wurden durchgeführt (IFCC: anerkannte Referenz für die Bestimmung von LDH enthaltend Lactat, NAD/NADH und N-Methylglucamin, pH 9.4; Eur. J. Clin. Chem. Biochem. vol. 32, p. 639-655 (1994)), Tabelle 1:

**Tabelle 1**

| Reagenz 1 | Reagenz 2 | Leerwert (LW) Unbelastet [mE/min] | Leerwert (LW) 5 Tage, 42°C [mE/min] | Signal Kalibrator -LW Unbelastet [mE/min] | Signal Kalibrator -LW 5 Tage 42°C [mE/min] |
|---|---|---|---|---|---|
| IFCC + 100 mmol/l Borat | IFCC | 1,3 | 6,3 | 32,4 | 31,0 = 95,7% |
| IFCC | IFCC + 100 mmol/l Citrat pH 3,0 | 2,1 | 6,2 | 35,2 | 33,4 = 95,4% |
| IFCC + 100 mmol/l Borat | IFCC + 100 mmol/l Citrat pH 3,0 | 1,0 | 3,0 | 30,9 | 29,4 = 95,1% |
| IFCC | IFCC + 50 mmol/l Hydroxylamin | 0,9 | 3,2 | 34,4 | 33,8 = 98,3% |
| IFCC + 100 mmol/l Borat | IFCC + 50 mmol/l Hydroxylamin | 0,7 | 1,8 | 31,1 | 30,5 = 98,0% |
| IFCC + 100 mmol/l Borat | IFCC + 50 mmol/ Hydroxylamin + 100 mmol/l Citrat pH 3,0 | 0,7 | 1,2 | 29,8 | 30,3 = 101,7% |
| IFCC (Stand der Technik) | IFCC | 1,5 | 11,5 | 35,7 | 33,7 = 94,5% |

| | | | | | |
|---|---|---|---|---|---|
| Ergebnis: Die erfindungsgemäße Rezeptur zeigt mit entsprechenden Zusätzen in Teilreagenz 1 und/oder Teilreagenz 2 insbesondere unter Belastung (5 Tage, 42°C) gegenüber der IFCC-Referenzmethode eine deutliche Verbesserung des Leerwertes, bei nahezu unveränderten Kalibrator-Leerwert. | | | | | |

### Beispiel 2

Es wurden die Ausgangslösungen wie in Beispiel 1 beschrieben verwendet und entsprechend verfahren. Reagenz 2 wurde Citrat und/oder verschiedene Hydroxylaminderivate in verschiedenen Konzentrationen und Kombinationen zugegeben (Tabelle 2).

**Tabelle 2**

| Reagenz 1 | Reagenz 2 | Leerwert (LW) Unbelastet [mE/min] | Leerwert (LW) 5 Tage, 42°C [mE/min] | Signal Kalibrator -LW Unbelastet [mE/min] | Signal Kalibrator -LW 5 Tage 42°C [mE/min] |
|---|---|---|---|---|---|
| IFCC = Referenz | IFCC = Referenz | 0,1 | 10,7 | 35,7 | 32,9 = 92,1% |
| IFCC | IFCC + 20 mmol/l Citrat + 50 mmol/l Hydroxylamin Sulfat | 0,8 | 2,5 | 31,6 | 31,2 = 98,7% |
| IFCC | IFCC + 20 mmol/l Citrat + 50 mmol/l Hydroxylamin Phosphat | 0,6 | 1,7 | 30,9 | 30,2 = 985,1% |
| IFCC | IFCC + 20 mmol/l Citrat + 50 mmol/l O-Benzylhydroxylamin | -0,9 | -0,6 | 34,8 | 33,3 = 95,7% |
| IFCC | IFCC + 20 mmol/l Citrat + 50 mmol/l O-Methylhydroxylamin | -0,7 | 0,0 | 35,2 | 33,6 = 95,5% |
| IFCC | IFCC + 20 mmol/l Citrat + 50 mmol/l N-Methylhydroxylamin | 4,9 | 11,1 | 34,6 | 34,6 = 100,0% |

| | | | | | |
|---|---|---|---|---|---|
| Ergebnis: Sämtliche erfindungsgemäß Reagenz 2 zugesetzten Verbindungen bzw. Salze resultieren in einer gegenüber dem IFCC-Reagenz verbesserten Wiederfindung nach Belastung (5 Tage, 42°C). | | | | | |

### Beispiel 3

Ferner wurde die Wiederfindung der verschiedenen Isoenzyme in der erfindungsgemäßen Rezeptur gezeigt. Diese muß der Wiederfindung der anerkannten IFCC-Empfehlung entsprechen (Tabelle 3).

Die Bestimmungen wurden mit dem in Beispiel 1 beschriebenen IFCC-Reagenz (Stand der Technik) im Vergleich zu einem erfindungsgemäßen Reagenz durchgeführt.

**Tabelle 3**

| Reagenz 1 | Reagenz 2 | Aktivität Isoenzym -1 [U/l] | Aktivität Isoenzym -2 [U/l] | Aktivität Isoenzym -3 [U/l] | Aktivität Isoenzym-4 [U/l] | Aktivität Isoenzym -5 [U/l] |
|---|---|---|---|---|---|---|
| IFCC | IFCC | 536 | 528 | 735 | 325 | 382 |
| IFCC + 100 mmol/l Borat | IFCC + 50 mmol/ Hydroxylamin + 100 mmol/l Citrat pH 3,0 | 536 | 523 | 736 | 301 | 368 |

| | | | | | | |
|---|---|---|---|---|---|---|
| Ergebnis: Die Wiederfindung der fünf LDH-Isoenzyme konnte mit dem erfindungsgemäßen Reagenz gezeigt werden. | | | | | | |

Werden die genannten Modifikationen an der Rezeptur vorgenommen, so kann ein LDH-Flüssigreagenz zur Verfügung gestellt werden, das während Lagerung (>12 Monate) und Transport (auch bei Temperaturen >8°C) stabil bleibt. Die sich daraus ergebenen Vorteile für den Anwender sind offensichtlich bzw. der zugrundeliegenden Beschreibung zu entnehmen.

## Patentansprüche

1. Stabilisierte wässrige Lösung eines Coenzyms für wasserstoffübertragende Enzyme, **dadurch gekennzeichnet, dass** die Lösung aus NAD, NADP oder einem entsprechenden Derivat in oxidierter oder reduzierter Form sowie einer oder mehreren organischen Verbindungen bzw. entsprechende Salze mit einem pKa-Wert zwischen 1,5 und 6,0 und Citronensäure bzw. Citratsalz und eine Stickstoffverbindung der allgemeinen Formel (I) 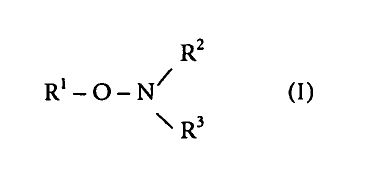 besteht, wobei der Rest R¹ Wasserstoff, eine gesättigte oder ungesättigte Alkyl- oder Arylgruppe bedeuten, und die Reste R² = R³ = H sind.

2. Stabilisierte Lösung nach Anspruch 1, **dadurch gekennzeichnet, dass** als organische Verbindung eine Säure bzw. ein entsprechendes Salz mit puffernder Wirkung im pH-Bereich von 1,0 bis 7,0 enthalten ist.

3. Stabilisierte Lösung nach Anspruch 1 oder 2, **dadurch gekennzeichnet, dass** ca. 5 bis 500 mM Citronensäure bzw. Citratsalz enthalten sind.

4. Stabilisierte Lösung nach einem der Ansprüche 1 bis 3, **dadurch gekennzeichnet, dass** der pH-Wert der Lösung zwischen 1,0 und 7,0 beträgt.

5. Stabilisierte Lösung nach einem der Ansprüche 1 bis 4 enthaltend ein Hydroxyl-, O- oder N-Alkylhydroxyl-, O-Benzylhydroxylamin.

6. Stabilisierte Lösung nach Anspruch 5, **dadurch gekennzeichnet, dass** ein Hydroxylaminderivat in einer Konzentration zwischen 2 und 300 mM enthalten sind.

7. Verfahren zur Bestimmung eines wasserstoffübertragenden Analyten oder einer entsprechenden Dehydrogenase in Gegenwart eines wasserstoffaufnehmenden Coenzyms, **dadurch gekennzeichnet, dass** das Coenzym in einer stabilisierten wässrigen Lösung gemäß der Ansprüche 1 bis 6 enthalten ist.

8. Verfahren zur Bestimmung nach Anspruch 7, **dadurch gekennzeichnet, dass** der Analyt Lactat, Glutamat, Ammoniak, Alkohol, Glycerinaldehyd-3-phosphat oder Glucose in Gegenwart einer Lactat-, Glutamat-, Alkohol-, Glycerin-3-phosphat- oder Glucose-Dehydrogenase bestimmt wird.

9. Verfahren nach einem der Ansprüche 7 oder 8, **dadurch gekennzeichnet, dass** der pH-Wert in einem Bereich von 8,5 bis 10,0 liegt und die Endkonzentration an Citratsalz und/oder Hydroxylaminderivat jeweils zwischen 2 und 50 mM beträgt.

10. Kit zur Bestimmung eines wasserstoffübertragenden Analyten in einer Probe bestehend aus folgenden Komponenten:
- einem ersten Reagenz, welches eine Dehydrogenase in einem geeigneten zwischen pH 8,5 und 10,0 puffernden System enthält,
und
- einem zweiten Reagenz gemäß Anspruch 1, welches ein Coenzym für wasserstoffübertragende Enzyme und eine organische Verbindung mit einem pKa-Wert zwischen 1,5 und 6,0 und Citronensäure bzw. Citratsalz und eine Stickstoffverbindung der allgemeinen Formel (I) 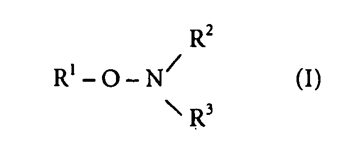 enthält, wobei der Rest R¹ Wasserstoff, eine gesättigte oder ungesättigte Alkyl- oder Arylgruppe bedeuten, und die Reste R² = R³ = H sind.

11. Kit zur Bestimmung der Enzymaktivität einer Dehydrogenase in einer Probe bestehend aus folgenden Komponenten:
- einem ersten Reagenz, welches einen wasserstoffübertragenden Analyten in einem geeigneten zwischen pH 8,5 und 10,0 puffernden System enthält,
und
- einem zweiten Reagenz gemäß Anspruch 1, welches ein Coenzym für wasserstoffübertragende Enzyme und eine organische Verbindung mit einem pKa-Wert zwischen 2,0 und 4,0 und Citronensäure bzw. Citratsalz und ein Hydroxylaminderivat der allgemeinen Formel (I) gemäß Anspruch 10 enthält.

12. Kit nach Anspruch 10 oder 11, **dadurch gekennzeichnet, dass** das zweite Reagenz Citronensäure, ein Citratsalz und Hydroxylaminderivat enthält.

13. Kit nach Anspruch 10 bis 12, **dadurch gekennzeichnet, dass** das zweite Reagenz einen pH-Wert zwischen 1,0 und 7,0 aufweist.

14. Kit nach einem der Ansprüche 10 bis 13, **dadurch gekennzeichnet, dass** das zweite Reagenz einen pH-Wert von etwa 3,0 aufweist.

15. Kit nach einem der Ansprüche 10 bis 14, **dadurch gekennzeichnet, dass** etwa 5 bis 200 mM eines Citratsalzes und/oder 2 bis 300 mM eines Hydroxylaminderivates im ersten oder zweiten Reagenz enthalten sind.

## Claims

1. Stabilized aqueous solution of a coenzyme for hydrogen-transferring enzymes, **characterized in that** the solution is composed of NAD, NADP or a derivative thereof in an oxidized or reduced form and one or more organic compounds or salts thereof having a pKa value between 1.5 and 6.0 and citric acid or a citrate salt and a nitrogen compound of the general formula (I) 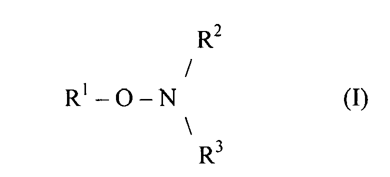 in which the residue R¹ denotes hydrogen or an unsaturated or saturated alkyl or aryl group, and the residues R² = R³ = H.

2. Stabilized solution according to claim 1, **characterized in that** it contains an acid or a salt thereof with a buffering action in the pH range of 1.0 to 7.0 as the organic compound.

3. Stabilized solution according to claim 1 or 2, **characterized in that** it contains about 5 to 500 mM citric acid or citrate salt.

4. Stabilized solution according to one of the claims 1 to 3, **characterized in that** the pH value of the solution is between 1.0 and 7.0.

5. Stabilized solution according to one of the claims 1 to 4 containing a hydroxyl, O- or N-alkyl-hydroxyl, O-benzylhydroxylamine.

6. Stabilized solution according to claim 5, **characterized in that** it contains a hydroxylamine derivative at a concentration between 2 and 300 mM.

7. Method for determining a hydrogen-transferring analyte or a corresponding dehydrogenase in the presence of a hydrogen-accepting coenzyme, **characterized in that** the coenzyme is contained in a stabilized aqueous solution according to claims 1 to 6.

8. Method for the determination according to claim 7, **characterized in that** the analyte lactate, glutamate, ammonia, alcohol, glyceraldehyde-3-phosphate or glucose is determined in the presence of a lactate, glutamate, alcohol, glycerol-3-phosphate or glucose dehydrogenase.

9. Method according to one of the claims 7 or 8, **characterized in that** the pH value is in a range of 8.5 to 10.0 and the final concentration of citrate salt and/or hydroxylamine derivative is in each case between 2 and 50 mM.

10. Kit for determining a hydrogen-transferring analyte in a sample comprising the following components:
- a first reagent which contains a dehydrogenase in a suitable system buffering between pH 8.5 and 10.0
and
- a second reagent according to claim 1 which contains a coenzyme for hydrogen-transferring enzymes and an organic compound having a pKa value between 1.5 and 6.0 and citric acid or a citrate salt and a nitrogen compound of the general formula (I) 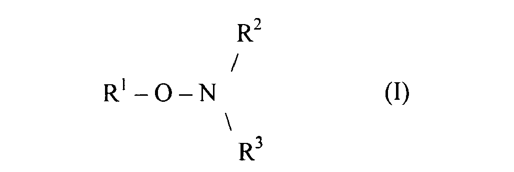
in which the residue R¹ denotes hydrogen, a saturated or unsaturated alkyl or aryl group, and the residues R² = R³ = H.

11. Kit for determining the enzyme activity of a dehydrogenase in a sample comprising the following components:
- a first reagent which contains a hydrogen-transferring analyte in a suitable system buffering between pH 8.5 and 10.0
and
- a second reagent according to claim 1 which contains a coenzyme for hydrogen-transferring enzymes and an organic compound having a pKa value between 2.0 and 4.0 and citric acid or a citrate salt and a hydroxylamine derivative of the general formula (I) according to claim 10.

12. Kit according to claim 10 or 11, **characterized in that** the second reagent contains citric acid, a citrate salt and a hydroxylamine derivative.

13. Kit according to claims 10 to 12, **characterized in that** the second reagent has a pH value between 1.0 and 7.0.

14. Kit according to one of the claims 10 to 13, **characterized in that** the second reagent has a pH value of about 3.0.

15. Kit according to one of the claims 10 to 14, **characterized in that** the first or second reagent contains approximately 5 to 200 mM of a citrate salt and/or 2 to 300 mM of a hydroxylamine derivative.

## Revendications

1. Solution aqueuse stabilisée d'un coenzyme pour enzymes de transfert d'hydrogène, **caractérisée en ce que** la solution se compose de NAD, NADP ou d'un dérivé correspondant sous une forme oxydée ou réduite ainsi que d'un ou de plusieurs composés organiques resp. sels correspondants avec une valeur de pKa comprise entre 1,5 et 6,0 et d'acide citrique resp. de sel de citrate et d'un composé d'azote dont la formule générale (I) est la suivante : 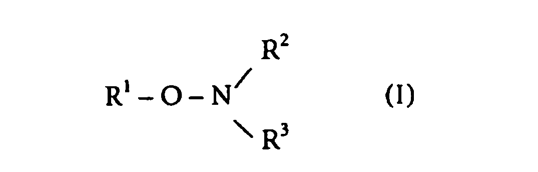 le reste R¹ correspondant à de l'hydrogène, un groupe alkyle ou aryle saturé ou non saturé et les restes R² et R³ étant tels que R² = R³ = H.

2. Solution stabilisée selon la revendication 1, **caractérisée en ce qu'**elle contient, en tant que composé organique, un acide resp. un sel correspondant à effet tampon dans la plage de pH de 1,0 à 7,0.

3. Solution stabilisée selon la revendication 1 ou 2, **caractérisée en ce qu'**elle contient environ 5 à 500 mM d'acide citrique resp. de sel de citrate.

4. Solution stabilisée selon l'une des revendications 1 à 3, **caractérisée en ce que** la valeur de pH de la solution est comprise entre 1,0 et 7,0.

5. Solution stabilisée selon l'une des revendications 1 à 4, contenant un hydroxylamine, O-alkyl hydroxylamine ou N-alkyl hydroxylamine, et un O-benzyl hydroxylamine.

6. Solution stabilisée selon la revendication 5, **caractérisée en ce qu'**elle contient un dérivé d'hydroxylamine dans une concentration comprise entre 2 et 300 mM.

7. Procédé pour déterminer un analyte de transfert d'hydrogène ou une déshydrogénase correspondant en présence d'un coenzyme absorbant l'hydrogène, **caractérisé en ce que** le coenzyme est contenu dans une solution aqueuse stabilisée selon les revendications 1 à 6.

8. Procédé de détermination selon la revendication 7, **caractérisé en ce que** l'analyte lactate, glutamate, ammoniaque, alcool, glycéraldéhyde 3-phosphate ou glucose est déterminé en présence d'une déshydrogénase de lactate, de glutamate, d'alcool, de glycérine 3-phosphate ou de glucose.

9. Procédé selon l'une des revendications 7 ou 8, **caractérisé en ce que** la valeur de pH est de l'ordre de 8,5 à 10,0 et la concentration finale de sel de citrate et/ou de dérivé d'hydroxylamine est respectivement comprise entre 2 et 50 mM.

10. Kit pour déterminer un analyte de transfert d'hydrogène dans un échantillon composé des composants suivants :
- un premier réactif qui contient une déshydrogénase dans un système approprié à effet tampon entre un pH de 8,5 et un pH de 10,0
et
- un deuxième réactif selon la revendication 1 qui contient un coenzyme pour des enzymes de transfert d'hydrogène et un composé organique avec une valeur de pKa comprise entre 1,5 et 6,0 et de l'acide citrique resp. un sel de citrate et un composé d'azote dont la formule générale (I) est la suivante : 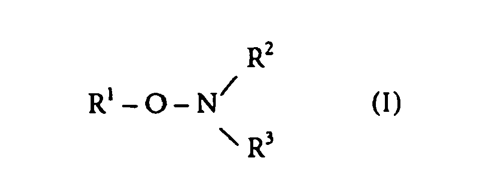 le reste R¹ correspondant à de l'hydrogène, un groupe alkyle ou aryle saturé ou non saturé et les restes R² et R³ étant tels que R² = R³ = H.

11. Kit pour déterminer l'activité enzymatique d'une déshydrogénase dans un échantillon composé des composants suivants :
- un premier réactif qui contient un analyte de transfert d'hydrogène dans un système approprié à effet tampon entre un pH de 8,5 et un pH de 10,0
et
- un deuxième réactif selon la revendication 1 qui contient un coenzyme pour des enzymes de transfert d'hydrogène et un composé organique avec une valeur de pKa comprise entre 2,0 et 4,0 et de l'acide citrique resp. un sel de citrate et un dérivé d'hydroxylamine dont la formule générale (I) est celle selon la revendication 10.

12. Kit selon la revendication 10 ou 11, **caractérisé en ce que** le deuxième réactif contient de l'acide citrique, un sel de citrate et un dérivé d'hydroxylamine.

13. Kit selon la revendication 10 à 12, **caractérisé en ce que** le deuxième réactif a une valeur de pH comprise entre 1,0 et 7,0.

14. Kit selon l'une des revendications 10 à 13, **caractérisé en ce que** le deuxième réactif a une valeur de pH d'environ 3,0.

15. Kit selon l'une des revendications 10 à 14, **caractérisé en ce que** le premier ou le deuxième réactif contiennent environ 5 à 200 mM d'un sel de citrate et/ou 2 à 300 mM d'un dérivé d'hydroxylamine.
